# FASCICULE DE BREVET EUROPEEN

(11) **EP 2 558 485 B1**
(45) Date de publication et mention de la délivrance du brevet: **22.02.2017**
(21) Numéro de dépôt: 11709481.3
(22) Date de dépôt: 16.02.2011
(51) Int. Cl.: C07K 14/435, A61K 39/00, C07K 16/18, G01N 33/68, G01N 33/569

(54) **ANTIGÈNES POLYPEPTIDIQUES DE TRICHINELLA, ET LEURS APPLICATIONS**
ANTIGENE TRICHINELLA-POLYPEPTIDE UND IHRE VERWENDUNG
ANTIGENIC POLYPEPTIDES OF TRICHINELLA AND USES THEREOF

(30) Priorité: 17.02.2010 FR 1000660
(43) Date de publication de la demande: 20.02.2013
(73) Titulaire: Agence nationale de sécurité sanitaire de l'alimentation,de l'environnement et du travail, 94701 Maisons-Alfort Cedex (FR)
(72) Inventeur: ZOCEVIC, Aleksandar, 93700 Drancy (FR); GIOVANI, Baldissera, 92320 Chatillon (FR); LACOUR, Sandrine A., 94140 Alfortville (FR); MACÉ, Pauline, 94100 Saint Maur Des Fossés (FR); VALLÉE, Isabelle, 94000 Créteil (FR); BOIREAU, Pascal, 91070 Bondoufle (FR)
(74) Mandataire: Gevers & Orès
(86) Numéro de dépôt international: PCT/IB2011/050645
(87) Numéro de publication internationale: WO 2011/101790

(56) Documents cités:
- WO-A1-2007/090960
- WO-A1-2009/003497
- GUILIANO D B ET AL: "Characterisation of novel protein families secreted by muscle stage larvae of Trichinella spiralis", INTERNATIONAL JOURNAL OF PARASITOLOGY, PERGAMON PRESS, GB LNKD- DOI:10.1016/J.IJPARA.2008.09.012, vol. 39, no. 5, 1 avril 2009 (2009-04-01), pages 515-524, XP026104798, ISSN: 0020-7519 [extrait le 2008-10-21] cité dans la demande -& DATABASE UniProt [Online] 16 décembre 2008 (2008-12-16), "SubName: Full=Secreted from muscle stage larvae 3;", XP002635638, extrait de EBI accession no. UNIPROT:B6SEV3 Database accession no. B6SEV3 -& DATABASE EMBL [Online] 5 novembre 2008 (2008-11-05), "Trichinella spiralis secreted from muscle stage larvae 3 (sml-3) mRNA, complete cds.", XP002635639, extrait de EBI accession no. EMBL:EU867516 Database accession no. EU867516
- WU X P ET AL: "Identification of antigenic genes in Trichinella spiralis by immunoscreening of cDNA libraries", VETERINARY PARASITOLOGY, ELSEVIER SCIENCE, AMSTERDAM, NL, vol. 159, no. 3-4, 23 février 2009 (2009-02-23), pages 272-275, XP025912018, ISSN: 0304-4017 [extrait le 2008-10-22] -& DATABASE UniProt [Online] 26 février 2008 (2008-02-26), "SubName: Full=Putative uncharacterized protein;", XP002635666, extrait de EBI accession no. UNIPROT:B0F9S7 Database accession no. B0F9S7
- MITREVA M ET AL: "Gene discovery in the adenophorean nematode Trichinella spiralis: an analysis of transcription from three life cycle stages", MOLECULAR AND BIOCHEMICAL PARASITOLOGY, ELSEVIER SCIENCE PUBLISHERS, AMSTERDAM, NL, vol. 137, no. 2, 1 octobre 2004 (2004-10-01), pages 277-291, XP004572709, ISSN: 0166-6851, DOI: DOI:10.1016/J.MOLBIOPARA.2004.05.015 -& DATABASE EMBL [Online] 15 juin 2002 (2002-06-15), "ps80f02.y1 Trichinella spiralis adult pAMP1 v1 Trichinella spiralis cDNA 5', mRNA sequence.", XP002635526, extrait de EBI accession no. EM_EST:BQ542776 Database accession no. BQ542776
- ROBINSON MARK W ET AL: "Proteomic analysis of the excretory-secretory proteins of the Trichinella spiralis L1 larva, a nematode parasite of skeletal muscle", PROTEOMICS, vol. 5, no. 17, novembre 2005 (2005-11), pages 4525-4532, XP002591964, ISSN: 1615-9853

## Description

La présente invention concerne l'utilisation de nouveaux antigènes identifiés chez le parasite *Trichinella* dans le cadre du diagnostic et de la prévention de la trichinellose.

La trichinellose est une zoonose associée à la consommation de viande infestée par le parasite *Trichinella* (MURRELL et al., Vet Parasitol, 93, 293-307, 2000).

Ce nématode, de la classe des *Adenophorea,* appartient à la famille des *Trichinellidae* laquelle comprend 8 espèces et 3 génotypes apparentés en 2 groupes phylogénétiquement distincts : d'une part les trichines encapsulées (*T. spiralis; T. nativa; T. britovi; T. murrelli; T. nelsoni*) qui infestent les mammifères, et d'autre part les trichines non-encapsulées (*T. pseudospiralis; T. papuae; T. zimbabwensis)* qui infestent les mammifères, les oiseaux et les reptiles (GASSER et al., Electrophoresis, 25, 3357-64, 2004). Toutes ces espèces peuvent infester l'homme.

Le cycle biologique du parasite est auto-hétéroxène : il se déroule entièrement chez un même hôte qui est à la fois hôte intermédiaire et hôte définitif (BOIREAU et al., Revue française des laboratoires, 71-89, 2002). Le passage des larves infestantes d'un hôte à un autre est nécessaire pour la réalisation d'un nouveau cycle. Ce passage se fait par ingestion de viande crue ou peu cuite contaminée par des larves du parasite. Au cours de la digestion, celles-ci sont libérées, et pénètrent dans l'épithélium intestinal où elles vont muer en vers adultes (Ad) sexués. Les femelles fécondées expulsent ensuite des larves L1 Nouveau-Nées (L1NN) qui gagnent les muscles striés via la circulation lymphatique et sanguine. Ces larves L1NN pénètrent dans les cellules musculaires (stade de développement infestant L1M : Larve L1 Musculaire) dont elles provoquent la dédifférenciation en cellules nourricières entourées d'une capsule de collagène protectrice épaisse dans le cas des trichines encapsulées, très fine dans le cas des trichines non-encapsulées.

Alors que la trichinellose est asymptomatique chez l'animal, l'infestation humaine se traduit durant la phase intestinale par des diarrhées associées à des nausées, vomissements et de violentes douleurs abdominales, alors que les symptômes liés à la phase d'invasion musculaire se caractérisent par l'association fièvre, oedème de la face et myalgies (CAPO & DESPOMMIER, Clin Microbiol Rev, 9, 47-54, 1996). Des atteintes oculaires, pulmonaires, gastro-intestinales, cardiaques et neurologiques peuvent venir compléter le tableau clinique de la trichinellose dont l'évolution peut être mortelle. Le caractère chronique de l'infestation, marqué par la persistance de douleurs musculaires chez les patients, est associé à la survie du parasite au sein de la cellule nourricière.

Le traitement spécifique des trichinelloses humaines par des anthelminthiques est d'autant plus efficace que le diagnostic de l'infestation est posé précocement pour permettre une action contre tous les stades parasitaires et surtout avant la formation de la capsule protectrice de collagène autour des larves L1M (FOURESTIE et al., Parasitol Res, 75, 36-41, 1988).

Les données épidémiologiques ont démontré une distribution géographique du parasite sous toutes les latitudes associée à un mode de transmission impliquant de nombreuses espèces de la faune sauvage qui entretiennent aussi un cycle d'infestation domestique principalement représenté par le porc (DUPOUY-CAMET, Vet Parasitol, 93, 191-200, 2000).

Les épidémies de trichinellose humaine, zoonose émergente ou ré-émergente, constituent un véritable problème de santé publique à travers le monde en raison des habitudes alimentaires et des contrôles sanitaires pas toujours efficaces (MURRELL & POZIO, Int J Parasitol, 30, 1339-49, 2000). Ces épidémies impliquent essentiellement la viande de porc et de sanglier ainsi que celle de cheval (BOIREAU et al., Vet Parasitol, 93, 309-20, 2000).

La prévention de la contamination humaine passe donc par une cuisson à coeur de la viande et l'amélioration des conditions d'élevage et/ou de contrôle des trichinelloses animales (porc, cheval, sanglier et autres espèces animales sauvages sensibles à *Trichinella*) (BOIREAU et al., Revue française des laboratoires, 71-89, 2002).

Les techniques de dépistage de la trichinellose se divisent en deux catégories : 1) la détection directe des larves L1M, après digestion artificielle d'échantillons de muscles, et 2) la détection indirecte par différentes méthodes immunologiques, permettant de détecter des anticorps dirigés contre les antigènes de *Trichinella.*

A chacun des stades de développement du parasite : Ad, L1NN, et L1M, correspond un profil antigénique particulier.

Ce sont des préparations antigéniques issues de larves du stade L1M qui sont actuellement utilisées pour l'immunodiagnostic. En effet, les fractions antigéniques des deux stades précoces Ad et L1NN sont difficiles à purifier, et des antigènes immunodominants associés à l'un et/ou l'autre de ces deux stades n'avaient pas pu être identifiés jusqu'à récemment (LIU *et al.,* 1-13,2007).

On utilise principalement soit des préparations d'antigène total soluble, obtenues par lyse des larves, centrifugation du lysat, et récupération du surnageant, soit plus fréquemment, des antigènes d'excrétion/sécrétion (antigènes E/S).

Les antigènes E/S sont produits lors de la mise en survie des larves L1M dans un milieu de culture ; ils proviennent d'un organe particulier, dénommé le stichosome, qui est constitué d'une cinquantaine de cellules discoïdes, les stichocytes. Les stichocytes contiennent des granules dont le contenu est évacué par un canalicule dans la lumière de l'oesophage du parasite. Ce contenu qui est très fortement antigénique, constitue une partie des antigènes E/S. Ces antigènes forment un mélange complexe de protéines, contenant notamment un groupe de glycoprotéines (dénommées antigènes TSL-1) portant une molécule glucidique particulière, connue uniquement chez *Trichinella* et présente chez toutes les espèces de ce parasite, le béta-tyvélose.

Les préparations d'antigènes E/S qui sont actuellement utilisées comme référence en matière d'immunodiagnostic de la trichinellose sont obtenues à partir de milieu de culture de larves L1M de *T. spiralis.* Après 18 à 20 heures de culture, le milieu est récupéré par filtration, puis concentré (GAMBLE et al., Vet Parasitol, 13, 349-61, 1983; GAMBLE et al., Vet Parasitol, 30, 131-7, 1988).

Les préparations d'antigène total soluble ont comme principal inconvénient leur manque de spécificité. Des réactions antigéniques croisées avec d'autres parasitoses sont fréquemment observées. Les antigènes E/S permettent d'obtenir une meilleure spécificité. Cependant, dans les deux cas, il est délicat de produire en quantité importante des lots standardisés d'antigène.

Un autre problème rencontré dans le cadre du diagnostic sérologique de *Trichinella* est l'existence d'une « fenêtre aveugle » de détection correspondant aux stades précoces de l'infestation, qui se traduit par des résultats sérologiques faussement négatifs. Cette fenêtre aveugle peut durer de 3 à 8 semaines, selon la dose infectieuse initiale. En outre, chez le cheval, une disparition progressive des anticorps a été observée 25 semaines après infestation.

Lors de travaux précédents, l'équipe des Inventeurs a identifié deux antigènes immunodominants conservés au sein du genre *Trichinella* et spécifiques de celui-ci. Ces antigènes sont décrits dans la demande PCT WO/2007/090960. L'un de ces antigènes, dénommé antigène NBL1, est exprimé spécifiquement au stade L1NN, tandis que l'autre, dénommé antigène 411, apparaît commun à plusieurs stades du développement de *Trichinella.*

Des tests de diagnostic sérologique de type ELISA ont été développés à partir de ces antigènes produits sous forme de protéines recombinantes purifiées. Ces tests permettent une détection sérologique très précoce des anticorps spécifiques anti-*Trichinella.* La comparaison avec un test ELISA de référence utilisant les antigènes E/S (test ELISA E/S) a permis de montrer que les tests ELISA utilisant les antigènes NBL1 et 411 peuvent détecter la présence d'anticorps spécifiques anti-*Trichinella* respectivement 5 à 45 jours plus tôt et 5 à 20 jours plus tôt que le test ELISA E/S. Cependant, la réponse humorale ciblant l'antigène NBL1 a ensuite tendance à s'atténuer (en moyenne 8 semaines après l'infestation), et l'antigène 411 présente l'inconvénient de présenter une sensibilité plus faible que le test ELISA E/S pour la détection d'une infestation par des espèces de *Trichinella* autres que *T. nativa.*

Les Inventeurs ont maintenant identifié deux nouveaux antigènes immunodominants de *Trichinella,* utilisables pour le développement de nouveaux tests de diagnostic sérologique, ou pour améliorer les performances des tests existants.

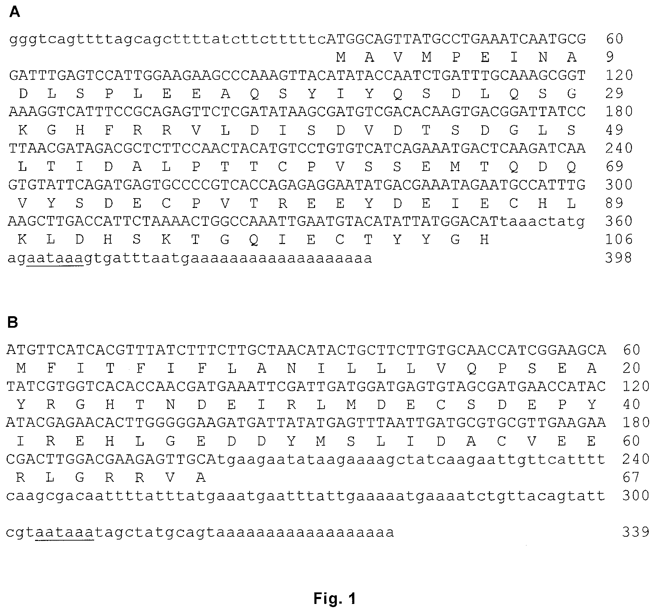

Le premier de ces antigènes est dénommé ci-après « L20h-Ts3 ». La séquence d'un clone d'ADNc codant pour cet antigène et la séquence polypeptidique déduite sont représentées sur la Figure 1A, et sont également respectivement reproduites dans la liste de séquences en annexe sous les numéros SEQ ID NO: 1 et SEQ ID NO: 2. Le polypeptide SEQ ID NO: 2 possède une identité élevée (91%) avec la protéine SML-3 (GenBank ACJ06741) récemment identifiée parmi les protéines vraisemblablement sécrétées par les larves de *T. spiralis* au stade musculaire, et qui pourrait jouer un rôle dans la formation de la cellule nourricière (GUILIANO et al., Int J Parasitol, 39, 515-24, 2009) ; cependant, les propriétés antigéniques de la protéine SML-3 n'ont pas été étudiées jusqu'à présent.

Le second antigène est dénommé ci-après « L20h-Ts1 ». La séquence d'un clone d'ADNc codant pour cet antigène et la séquence polypeptidique déduite sont représentées sur la Figure 1B, et sont aussi respectivement reproduites dans la liste de séquences en annexe sous les numéros SEQ ID NO: 3 et SEQ ID NO: 4. Le polypeptide SEQ ID NO: 4 ne possède d'identité avec aucune protéine connue de *Trichinella* et d'autres organismes.

Les polypeptides L20h-Ts3 et L20h-Ts1, produits sous forme recombinante, permettent de détecter à un stade précoce, la réponse humorale dirigée contre *Trichinella,* En outre, la réponse humorale ciblant ces antigènes persiste plus tardivement après l'infestation que dans le cas de l'antigène recombinant NBL1, où elle commence à s'atténuer approximativement 8 semaines après l'infestation. L'utilisation des polypeptides L20h-Ts3 et L20h-Ts1 permet donc d'élargir la fenêtre temporelle de détection de la réponse humorale.

La présente invention a en conséquence pour objet l'utilisation d'un polypeptide antigénique reconnu par des anticorps anti-*Trichinella* comme réactif pour la détection d'anticorps *anti-Trichinella* dans un prélèvement biologique, caractérisée en ce que ledit polypeptide est un polypeptide a) comprenant la séquence SEQ ID NO: 2, ou comprenant une séquence présentant au moins 95% d'identité avec la séquence SEQ ID NO: 2.

Avantageusement, dans le cadre de la mise en oeuvre de la présente invention, on peut utiliser un mélange, comprenant un polypeptide a) tel que défini ci-dessus et un polypeptide b) comprenant les acides aminés 21-67 de la séquence SEQ ID NO: 4 (qui représentent la forme mature de la protéine L20h-Tsl), ou comprenant une séquence présentant au moins 95% d'identité avec la séquence des acides aminés 21-67 de la séquence SEQ ID NO: 4, ou bien un mélange comprenant un polypeptide a) et un polypeptide b) tels que définis ci-dessus, associé(s) avec un ou plusieurs autre(s) polypeptide(s) antigénique(s) reconnu(s) par des anticorps anti-*Trichinella,* par exemple un ou plusieurs des polypeptides issus des antigènes NBL1 et 411, décrits dans la demande PCT WO/2007/090960.

La présente invention a plus particulièrement pour objet un procédé de détection de la présence d'anticorps *anti-Trichinella* dans un échantillon biologique, lequel procédé est caractérisé en ce qu'il comprend :
- la mise en contact dudit échantillon biologique avec un polypeptide a) ou un polypeptide a) et un polypeptide b), tels que définis ci-dessus, dans des conditions permettant la formation d'un complexe antigène/anticorps entre le(s)dit(s) polypeptide(s) et les anticorps anti-*Trichinella* éventuellement présents dans ledit échantillon ;
- la détection, par tous moyens appropriés, du complexe antigène/anticorps éventuellement formé.

Généralement, ledit prélèvement biologique est un prélèvement de sérum. Il peut être obtenu à partir de tout sujet (mammifères, oiseau ou reptile) appartenant à une espèce pouvant être infestée par *Trichinella,* et chez lequel on souhaite détecter la présence de ce parasite. Avantageusement, il s'agit d'un prélèvement obtenu à partir d'un mammifère, par exemple d'un animal d'élevage, ou d'un patient humain.

Cette association permet notamment d'élargir le spectre de réactivité, ainsi que la fenêtre temporelle de détection de la réponse humorale, par rapport à chacun des polypeptides utilisés individuellement.

La présente invention englobe notamment des polypeptides chimériques comprenant une ou plusieurs copies d'un polypeptide a) et une ou plusieurs copies d'un polypeptide b) ou plusieurs copies d'un polypeptide a) tels que définis ci-dessus, éventuellement fusionnés à un ou plusieurs polypeptide(s) hétérologue(s), par exemple un ou plusieurs des polypeptides issus des antigènes NBL1 et 411, décrits dans la demande PCT WO/2007/090960.

La présente invention a également pour objet les polynucléotides codant pour un polypeptide chimérique conforme à l'invention, ainsi que des vecteurs recombinants comprenant lesdits polynucléotides, et des cellules-hôtes transformées par lesdits vecteurs.

Les polypeptides a) et b) définis ci-dessus peuvent être utilisés dans le cadre de différentes méthodes de détection d'anticorps, qui sont connues en elles-mêmes. A titre d'exemples, on citera notamment les méthodes de type ELISA (direct, indirect ou sandwich), les méthodes de micro agglutination sur billes, ainsi que les méthodes de transfert électrophorétique couplé à un immunomarquage.

La présente invention a également pour objet un nécessaire pour la détection de la présence d'anticorps anti-*Trichinella* dans un échantillon biologique, caractérisé en ce qu'il comprend un polypeptide a) ou un polypeptide a) et un polypeptide b) tels que définis ci-dessus, et, le cas échéant, des tampons et réactifs appropriés pour la constitution d'un milieu réactionnel permettant la formation d'un complexe antigène/anticorps, et, optionnellement, des moyens de détection dudit complexe antigène/anticorps. Éventuellement il peut comprendre un ou plusieurs autres polypeptides antigéniques reconnus par des anticorps anti-*Trichinella,* par exemple un ou plusieurs des polypeptides issus des antigènes NBL1 et 411, décrits dans la demande PCT WO/2007/090960.

Avantageusement, le ou lesdits polypeptide(s) est (sont), immobilisé(s) sur un support solide. A titre d'exemples non-limitatifs de supports solides utilisables, on citera des plaques de microtitration, des billes, des microbilles ou microparticules, des bandelettes, etc....

Ledit nécessaire peut également comprendre des échantillons de référence, tels qu'un ou plusieurs sérum(s) négatif(s) et un ou plusieurs sérum(s) positif(s).

La présente invention sera mieux comprise à l'aide du complément de description qui va suivre, qui se réfère à des exemples illustrant l'utilisation des antigènes L20h-Ts3 et L20h-Ts1, pour l'immunodiagnostic de la trichinellose.

### EXEMPLE 1 : IDENTIFICATION DES ANTIGENES L20H-TS3 ET L20H-TS1, ET PRODUCTION DE CES ANTIGENES SOUS FORME DE PROTEINES RECOMBINANTES EN SYSTEME D'EXPRESSION PROCARYOTE.

Un immunocriblage de banques d'ADNc d'expression des stades précoces de *Trichinella* (14h, 20h, 48h après infection de souris) a été effectué avec des sérums et des surnageants de culture de muqueuse intestinale de porcs expérimentalement infestés par *T. spiralis.*

Le gène L20h-Ts3 a été identifié dans 41 des clones des banques d'ADNc obtenues 14h et 20h après infection des souris. La séquence nucléotidique et la séquence polypeptidique déduite de l'un de ces clones sont représentées sur la Figure 1A.

Sa longueur est de 398 pb, dont 33 pb de 3'UTR, un signal de polyadénylation putatif (souligné sur la Figure 1A), et un cadre ouvert de lecture codant pour une protéine de 106 a.a., avec une masse moléculaire de 11906,0 Da et un point isoélectrique de 4,21. Aucun peptide signal n'a été identifié sur la base de l'algorithme de BENDTSEN *et al.* (BENDTSEN et al., J Mol Biol, 340, 783-95, 2004). La recherche d'homologues sur les bases de données fait apparaître une forte identité (91%) entre la séquence polypeptidique déduite de L20h-Ts3 et une protéine de *T. spiralis,* dénommée SML-3 (GUILIANO et al., Int J Parasitol, 39, 515-24, 2009).

Ce clone a été digéré par *Bam*HI-*Not*I (New England BioLabs, Beverly, Massachusetts), et le fragment de restriction contenant la séquence codante complète de L20h-Ts3 a été sous-cloné dans le vecteur pGEX-6P-1 (GenBank U78872). Ce vecteur possède une étiquette Glutathion S-transférase de *Schistosoma japonicum* (GST*sj*) en N-terminal (La GST*sj* ne présente pas de réactions immunologiques croisées avec *Trichinella*). Le vecteur d'expression résultant, dénommé pGEX-6P-1-(L20h-Ts3), code pour une protéine recombinante contenant la totalité de la séquence de L20h-Ts3 et portant en position N-terminale une étiquette GST*sj* et en position C-terminale une étiquette poly-histidine.

Le gène L20h-Ts1 a été identifié dans 121 des clones des banques d'ADNc obtenues 20h et 48h après infection des souris. La séquence de l'un de ces clones est représentée sur la Figure 1B. Sa longueur est de 339 pb, dont 139 pb de 3'UTR, un signal de polyadénylation putatif (souligné sur la Figure 1B), et un cadre ouvert de lecture codant pour une protéine de 67 a.a., avec une masse moléculaire de 7834,9 Da et un point isoélectrique de 4,42. Un peptide signal de 20 a.a. (en gras sur la Figure 1B) a été identifié sur la base de l'algorithme de BENDTSEN *et al.* (2004). La recherche d'homologues sur les bases de données n'a fait apparaître aucune homologie avec des protéines connues de *Trichinella.*

Ce clone a été digéré par *Bam*HI-*Not*I (New England BioLabs, Beverly, Massachusetts), et un fragment de restriction codant pour le fragment Tyr₂₁-Ala₆₇ de L20h-Ts1 (correspondant à la forme mature, sans le peptide signal) a été sous-cloné dans le vecteur pGEX-6P-1. Le vecteur d'expression résultant dénommé pGEX-6P-1-(L20h-Ts1) code pour une protéine recombinante contenant la séquence de la forme mature de L20h-Ts1 et portant en position N-terminale une étiquette GST*sj* et en position C-terminale une étiquette poly-histidine.

Les vecteurs pGEX-6P-1-(L20h-Ts3) ou pGEX-6P-1-(L20h-Ts1) ont été utilisés pour transformer des bactéries *E. coli* (souche BL21), et l'expression de chaque protéine recombinante (contenant un tag GST*sj* en N-Ter et un tag Histidine en C-Ter) a été induite par ajout d'isopropyl-β-D-thiogalactopyranoside (IPTG) 0,5 mM (concentration finale) et incubation 3h, à 37°C et 225 tr/min. Les bactéries induites ont été centrifugées (4000 x g, 20 min à 4°C), resuspendues en tampon de lyse (Tris-HCl 20 mM pH 8,0; NaCl 150mM) supplémenté en lysozyme 0,5 mg/mL, et lysées par trois cycles de congélation/décongélation en azote liquide.

Ensuite, 5 µg/mL de DNase I ont été ajoutés au lysat incubé 15 min à 37°C. Puis, le lysat a été incubé pendant 1h30 à 4°C sur agitateur rotatif dans un tube Falcon® à fond conique de 50 mL (Becton Dickinson, Le Pont-De-Claix, France) en présence d'une matrice Ni-NTA (GE Healthcare Europe, Orsay, France) pré-équilibrée en tampon de lyse. Le mélange lysat-matrice a été déposé sur une colonne PD-10 (GE Healthcare Europe, Orsay, France) afin d'éliminer les protéines non-fixées ou les contaminants, puis transféré dans un nouveau tube Falcon® de 50 mL et lavé avec 50 mL de tampon de lavage I (Tris-HCl 20 mM pH 8.0; NaCl 300 mM) pendant 30 min à 4°C sur agitateur rotatif. Après centrifugation pendant 5 min (1500 x g, 4°C), le mélange a été lavé 4 fois avec 50 mL de tampon de lavage II (Tris-HCl 20 mM pH 8.0; NaCl 300 mM; imidazole 30 mM) pendant 30 min à 4°C ; ces lavages ont été suivis d'une ultime centrifugation pendant 5 min (1500 x g, 4°C). Enfin, chaque protéine recombinante a été éluée avec le tampon d'élution (Tris-HCl pH 8.0 20 mM; NaCl 300 mM; imidazole 500 mM) et la concentration déterminée à l'aide d'un spectrophotomètre à 280 nm. La protéine recombinante a été mise en fractions aliquotes et conservée à -20°C en glycérol 10 %.

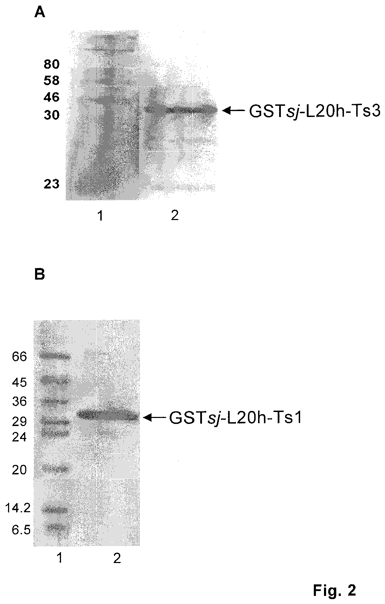

Les gels d'électrophorèse en conditions dénaturantes (SDS-PAGE) des protéines de fusion GST*sj*-L20h-Ts3 et GST*sj*-L20h-Ts1 sont respectivement représentés sur les Figures 2A et 2B (piste 1 : marqueurs de poids moléculaire). Sur ces gels, les protéines GSTsj-L20h-Ts3 et GST*sj*-L20h-Ts1 apparaissent toutes deux sous la forme d'une seule bande à la taille attendue pour la protéine de fusion.

### EXEMPLE 2 : IMMUNOREACTIVITE DES PROTEINES L20H-TS3 ET L20H-TS1 EN TRANSFERT DE WESTERN.

### Sérums anti-Trichinella

Des porcs Exempts d'Organismes Pathogènes Spécifiés (porcs EOPS) ou des porcs issus d'un élevage conventionnel ont été infestés avec 200, 1000, 20000 larves infestantes de *T. spiralis* (ISS004, International Trichinella Reference Centre, Rome, Italie) ou de *T. britovi* (ISS100). Des prélèvements sanguins ont été réalisés 48h avant infestation (témoin négatif), et à 5, 10, 15, 20, 25, 30, 40, 50, 60 jours post-infestation (pi).

Des sérums provenant de porcs EOPS infestés avec 20000 larves L1M de *T. spiralis* (ISS003) ou *T. britovi* (ISS002) ont également été utilisés. Des prélèvements sanguins ont été réalisés 24h avant infestation (témoin négatif), et à 1, 2, 3, 4, 5, 6, 7, 8, 12, 16, 20, 25 semaines pi.

Des sérums de porcs provenant d'élevages industriels hors-sol d'Ille-et-Vilaine (France), et contrôlés négatifs pour la trichinellose par digestion artificielle, ont été collectés en 2004 et ont servi de sérums de référence négatifs.

### Transfert de Western

L'immunoréactivité des antigènes L20h-Ts3 et L20h-Ts1 recombinants (sous forme des protéines de fusion GST*sj*-L20h-Ts3 et GST*sj*-L20h-Ts1) a été analysée par transfert de Western.

Les protéines recombinantes ont été soumises à une électrophorèse en conditions dénaturantes (15 % SDS-PAGE, 700 ng de protéine par puits), et transférées sur membrane de nitrocellulose. Après blocage des membranes par incubation pendant 1 nuit à 4°C dans du tampon PBS contenant 0,1% de Tween®-20(PBS-T) et 5 % de lait écrémé, celles-ci ont été lavées 3 fois en PBS-T pendant 5 min. Ensuite, les membranes ont été incubées pendant 1 h à température ambiante avec :
- dans le cas de L20h-Ts3, des sérums de porcs EOPS expérimentalement infestés avec 20000 larves L1M de *T. spiralis,* collectés à -2, 5, 12, 15, 20, 28, et 60 jours après infestation et dilués au 1/300 en PBS-T contenant 5 % de lait écrémé,
- dans le cas de L20h-Ts1, le sérum d'un porc EOPS expérimentalement infesté avec 20000 larves L1M *de T. spiralis,* collectés à -1, 7, 14, 21, 28, 49, 84 jours après infestation et dilués au 1/300 en PBS-T contenant 5 % de lait écrémé.

Après 3 lavages de 5 min en PBS-T, les membranes ont été incubées pendant 1h à température ambiante avec un anticorps secondaire de lapin anti-IgG de porc marqué à la peroxydase dilué au 1/10000. Après 3 nouveaux lavages, la révélation a été effectuée à l'aide du kit « ECL PLUS WESTERN BLOTTING ET AMERSHAM HYPERFILM ECL » (GE Healthcare Europe, Orsay, France).

### L20h-Ts3 :

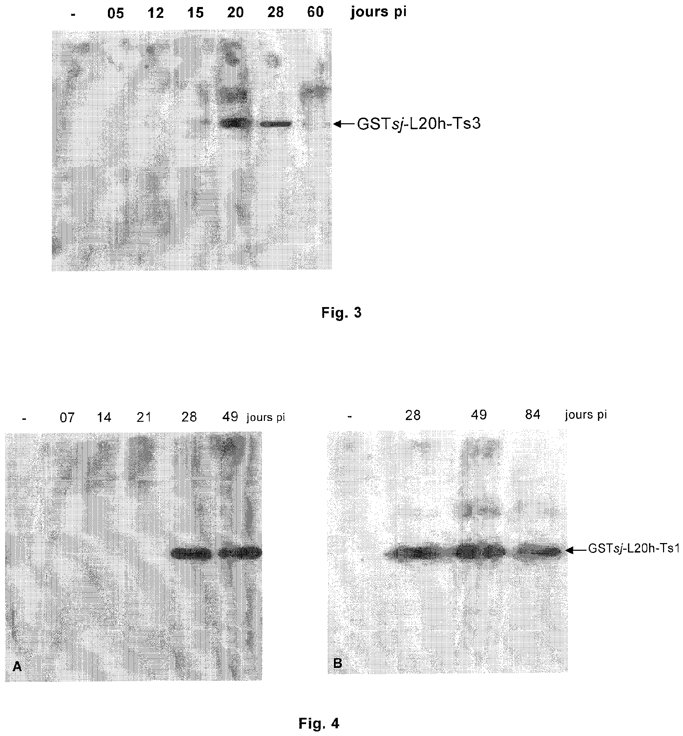

Les résultats obtenus avec les sérums de porcs EOPS expérimentalement infestés avec 20000 larves L1M de *T. spiralis* et collectés à -2, 5, 12, 15, 20, 28, et 60 jours pi sont illustrés par la Figure 3. Ces résultats montrent que l'antigène recombinant L20h-Ts3 (GST*sj*-L20h-Ts3) est nettement reconnu dès 20 jours pi (une faible bande est visible dès 15 jours pi), et au moins jusqu'à 60 jours pi.

### L20h-Ts1 :

Les résultats sont illustrés par la Figure 4. Ces résultats montrent que l'antigène recombinant L20h-Ts1 (GST*sj*-L20h-Ts1) est nettement reconnu dès 28 jours pi (une faible bande est visible dès 21 jours pi), et au moins jusqu'à 84 jours pi par le sérum d'un porc EOPS expérimentalement infesté avec 20000 larves L1M de *T. spiralis* (Figures 4A et 4B) (Il est à noter qu'une détection à 112 jours pi a également été possible avec un autre porc EOPS expérimentalement infesté avec 20000 larves L1M *T. spiralis,* donnée non montrée). L'antigène recombinant L20h-Ts1 (GST*sj*-L20h-Ts1) est également reconnu (une faible bande est visible) à 60 jours pi dans le cas d'un porc infesté avec 200 larves L1M de *T. spiralis* (donnée non montrée).

### EXEMPLE 3: IMMUNOREACTIVITE EN ELISA INDIRECT DE L'ANTIGENE RECOMBINANT L20H-TS3

L'immunoréactivité de l'antigène recombinant L20h-Ts3 a été évaluée par ELISA indirect, par comparaison avec l'antigène E/S de *T. spiralis,* et avec l'antigène NBL1 décrit dans la Demande PCT WO/2007/090960.

Les sérums issus de porcs infestés par *Trichinella* utilisés sont ceux décrits à l'Exemple 2 ci-dessus. Pour la détermination du seuil de diagnostic, un total de 220 sérums de références de porcs négatifs a été utilisé.

L'antigène E/S utilisé comme référence est celui du test ELISA commercialisé par l'Institut Pourquier (Montpellier, France).

Le protocole utilisé pour les tests ELISA est le suivant : 100 µL/puits d'antigène dilué en tampon de sensibilisation (carbonate de sodium 12 mM; bicarbonate de sodium 35 mM, pH 9,6) ont été déposés et incubés pendant 2h à 37°C dans une plaque de microtitration (Immuno 96 MicroWell Plates F96 MaxiSorp, Nunc, Roskil, Danemark). Ensuite, chaque puits a été lavé 5 fois avec 250 µL de tampon de lavage (eau distillée, Tween®-20 0,05 %) et incubé pendant 30 min à 37°C avec 200 µL de tampon de saturation (PBS, gélatine 1 %; Tween®-20 0,05 %). Après 5 lavages avec la solution de lavage, 200 µL/puits d'anticorps primaires (sérums) dilué en tampon de saturation ont été déposés et incubés 1h à 37°C. Après 5 lavages avec la solution de lavage, 100 µL/puits d'anticorps secondaires de lapin anti-IgG de porc conjugués à la peroxydase (dilué au 1/50000 en tampon de saturation) ont été déposés et incubés pendant 1h à 37°C. Enfin, les puits ont été lavés 5 fois avec la solution de lavage et 100 µL/puits de 3, 3', 5, 5' TetraMethylBenzidine (TMB, Zymed, California, USA) ont été utilisés pour la révélation en chambre noire. La réaction a été arrêtée avec 50 µL/puits d'acide sulfurique 12,5 % et la lecture faite à 450 nm à l'aide d'un lecteur de plaque Labsystems iEMS Reader MF couplé au logiciel Ascent 2.6 (Thermo LabSystems, Cergy Pontoise, France).

Les conditions optimales pour l'ELISA ont été définies en testant des échantillons de sérum à des dilutions de 1/10, 1/100 et 1/300, et des quantités de protéine L20h-Ts3 de 50 ng, 250 ng, 500 ng and 1 µg par puits. Les résultats obtenus ont ensuite été analysés par l'intermédiaire d'un test de Wilcoxon-Mann-Whitney. Ainsi, la différence la plus importante entre les valeurs des échantillons positifs et les valeurs des échantillons négatifs a été observée pour une dilution de 1/10 des sérums et 250 ng de protéine L20h-Ts3. Ces conditions ont donc été utilisées lors des ELISA ultérieurs.

Ces conditions ont donc été utilisées pour la suite des tests. Le seuil de diagnostic a été déterminé à partir de sérums négatifs de référence. Le calcul repose sur l'utilisation de la formule définie par l'Office International des Epizooties (Manuel des tests de diagnostic et des vaccins pour les animaux terrestres, 6e éd., 2008, Chapitre 2.01.6), correspondant à la moyenne des D.O. mesurées à 450 nm à laquelle on ajoute 3 fois la déviation standard.

Les résultats sont illustrés par les Figures 5 à 13.
La Figure 5 représente les valeurs de densités optiques mesurées sur 220 sérums négatifs de référence. Le seuil diagnostic établi à partir de ces mesures est de 0,33 unités de D.O.
La Figure 6 représente les valeurs de densité optique mesurées avec des sérums de porcs EOPS (▲) ou des porcs issus d'un élevage conventionnel (■), infestés avec 20000 larves L1M de *T. spiralis,* pour des sérums collectés 5 (porcs 1-3), 12 (porcs 4-6), 15 (porcs 7-9), 20 (porcs 10-12) et 60 (porcs 13-15) jours pi. La fenêtre aveugle, pendant laquelle aucune détection n'est possible avec l'ELISA E/S, est représentée en grisé.
La Figure 7 représente l'évolution de la densité optique (en ordonnée) au cours des semaines suivant l'infestation (en abscisse) pour les sérums de 3 porcs EOPS expérimentalement infestés avec 20000 larves L1M de *T. spiralis* et testés avec l'antigène L20h-Ts3.
La Figure 8 représente l'évolution de la densité optique (en ordonnée) au cours des semaines suivant l'infestation (en abscisse) pour les sérums de 3 porcs EOPS expérimentalement infestés avec 20000 larves L1M de *T. spiralis* et testés avec l'antigène E/S. Le % E/P est défini par la formule suivante : (D.O. de l'échantillon à tester) / (D.O. moyenne de l'échantillon de contrôle positif) x 100.
La Figure 9 représente l'évolution de la densité optique (en ordonnée) au cours des semaines suivant l'infestation (en abscisse) pour les sérums de 3 porcs EOPS expérimentalement infestés avec 20000 larves L1M de *T. spiralis* et testés avec l'antigène NBL1.
La Figure 10 représente l'évolution de la densité optique (en ordonnée) au cours des semaines suivant l'infestation (en abscisse) pour les sérums de 3 porcs EOPS expérimentalement infestés avec 20000 larves L1M de *T. britovi* et testés avec l'antigène L20h-Ts3.
La Figure 11 représente l'évolution de la densité optique (en ordonnée) au cours des semaines suivant l'infestation (en abscisse) pour les sérums de 3 porcs EOPS expérimentalement infestés avec 20000 larves L1M de *T. britovi* et testés avec l'antigène E/S.
La Figure 12 représente l'évolution de la densité optique (en ordonnée) au cours des semaines suivant l'infestation (en abscisse) pour les sérums de 3 porcs EOPS expérimentalement infestés avec 20000 larves L1M de *T. britovi* et testés avec l'antigène NBL1.
La Figure 13 représente les valeurs de densité optique mesurée avec des sérums de porcs EOPS (▲) ou de porcs issus d'un élevage conventionnel (■), infestés avec 200 (porcs 1-3) ou 1000 (porcs 4-6) larves L1M de *T. spiralis* et testés avec l'antigène L20h-Ts3.

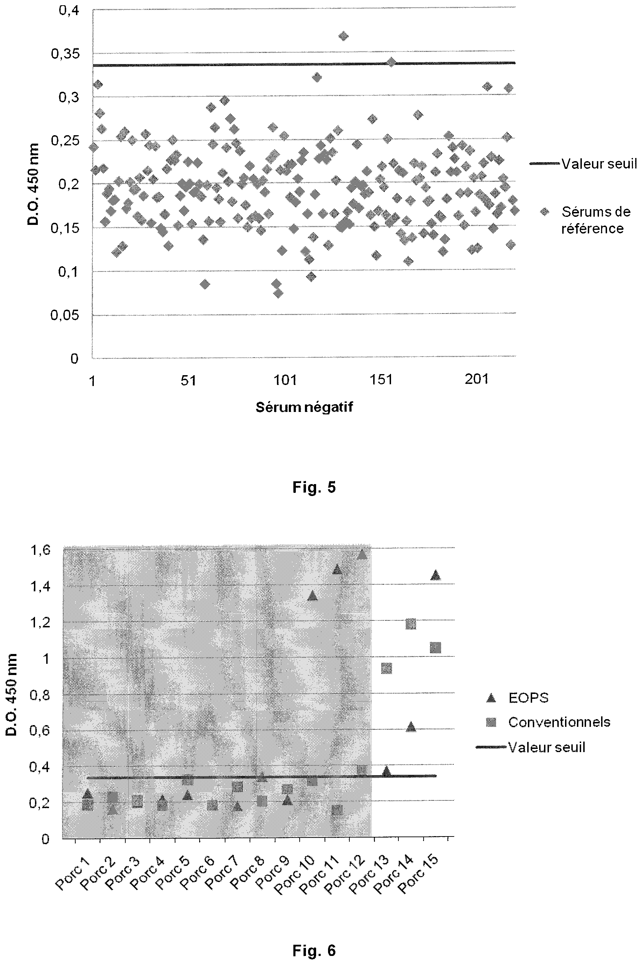

Dans le cas de porcs expérimentalement infestés avec 20000 L1M *T. spiralis,* la détection avec l'antigène L20h-Ts3 était possible dès 15 jours pi pour 1/6 porc (porc 8) et 20 jours pi pour 4/6 porcs (Figure 6). L'ELISA E/S n'a quant à lui pas permis une détection de l'infection avant 25 jours. Les résultats obtenus étaient différents selon le statut sanitaire des porcs. En effet, les porcs EOPS ont été détectés de façon plus précoce par rapport aux porcs conventionnels.

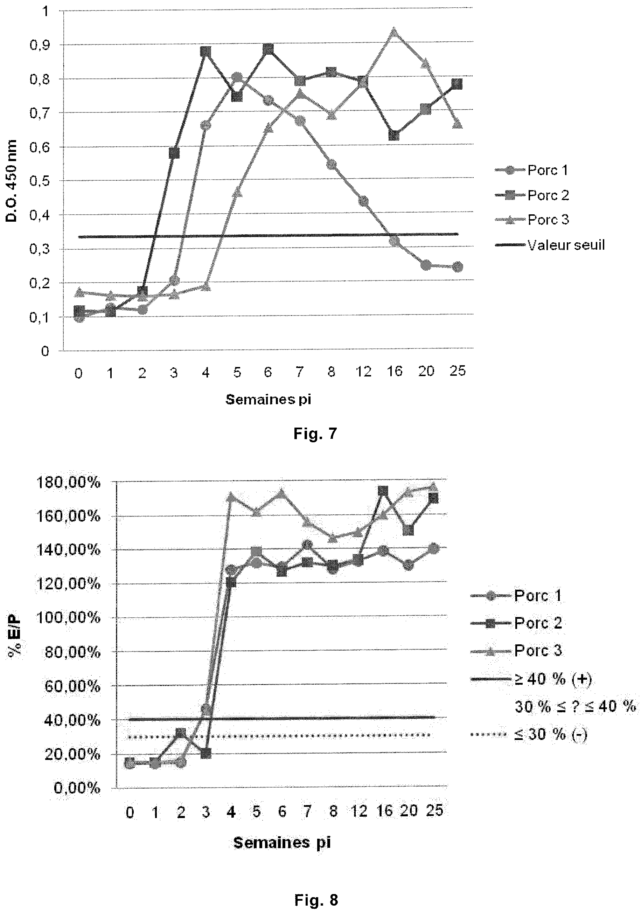

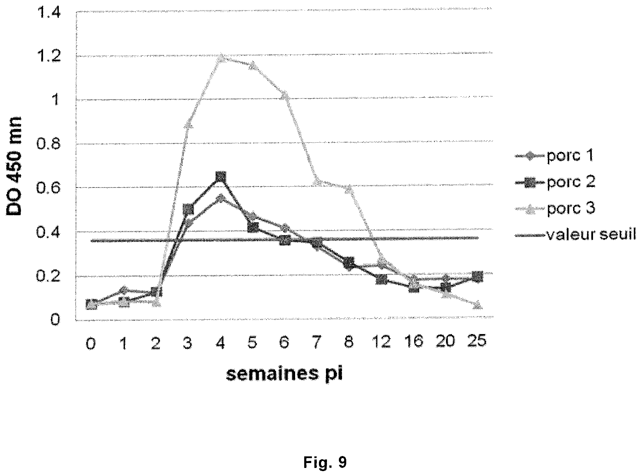

La séroconversion s'est accompagnée d'un profil de réponses humorales ayant des titres élevés et maintenus jusqu'à 25 semaines pi pour 2/3 porcs et jusqu'à 12 semaines pi pour 1/3 porc infestés avec *T. spiralis* (Figure 7). Un porc sur trois a été détecté plus précocement (1 semaine plus tôt) qu'avec l'ELISA E/S (Figure 8). Sur les 3 mêmes animaux, l'antigène NBL1 (Figure 9) a permis une détection d'anticorps *anti-Trichinella* dès 3 semaines pi. En revanche, une chute de réponse est observée à 7 semaines pi pour 2 de ces 3 porcs. Pour le troisième porc, la présence d'anticorps anti-*Trichinella* n'est plus détectée après 12 semaines.

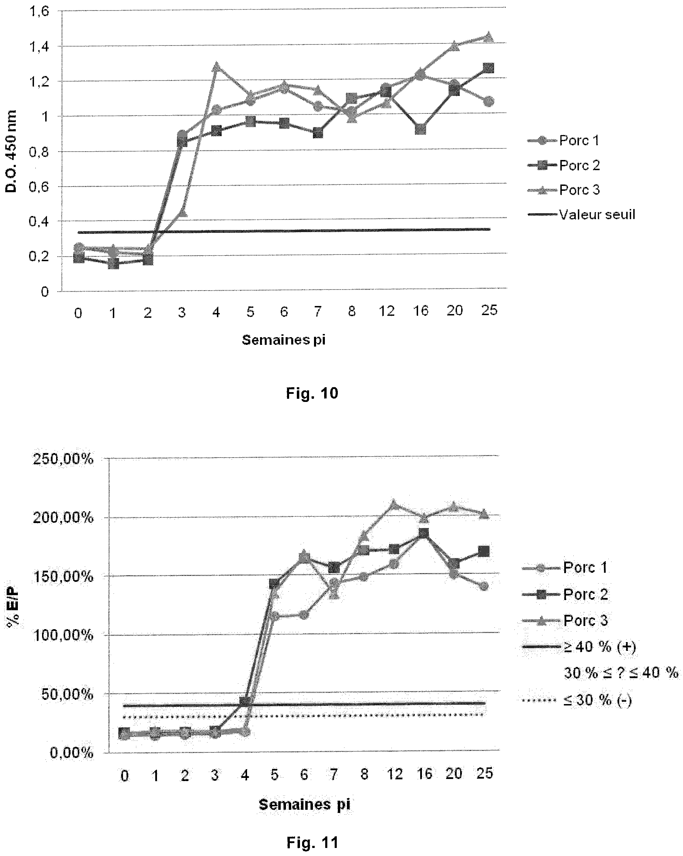

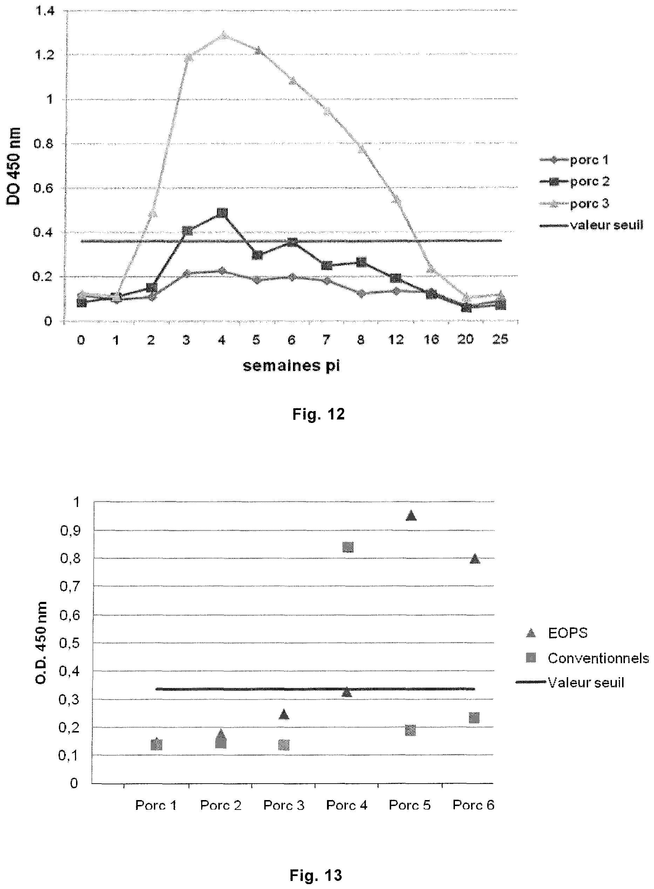

Dans le cas de porcs expérimentalement infestés avec 20000 L1M *T. britovi,* la séroconversion a été observée une à deux semaines plus précocement avec l'antigène L20h-Ts3 qu'avec l'ELISA E/S (Figures 10 et 11) avec des D.O. mesurées élevées dès 3 semaines pi et maintenues jusqu'à 25 semaines pi pour les trois porcs testés. Sur les 3 mêmes animaux, l'antigène NBL1 (Figure 12) a permis la détection des anticorps anti-*Trichinella* dans le sérum de l'un des porcs dès 2 semaines pi et jusqu'à 16 semaines pi. La présence d'anticorps anti*Trichinella* a été détectée dans le sérum d'un second porc uniquement les troisième et quatrième semaines pi. Enfin, aucun anticorps anti-*Trichinella* n'a été détecté dans le sérum du troisième porc.

De plus, 3/6 porcs avec une dose infestante modérée (1000 L1M, *T. spiralis*) ont été détectés à 60 jours pi avec l'antigène L20h-Ts3 (Figure 13). De nouveau, les résultats obtenus dépendaient du statut sanitaire des porcs. En effet, 2/3 porcs EOPS et 1/3 porc conventionnel ont été détectés. Les porcs infestés avec une dose infestante faible (200 L1M, *T. spiralis)* n'ont pas été détectés à 60 jours pi.

## Revendications

1. Utilisation d'un polypeptide antigénique comme réactif pour la détection d'anticorps anti-*Trichinella* induits par l'infestation par *Trichinella* dans un prélèvement biologique, **caractérisée en ce que** ledit polypeptide comprend la séquence SEQ ID NO: 2 ou une séquence présentant au moins 95% d'identité avec la séquence SEQ ID NO: 2 et ledit polypeptide étant reconnu par des anticorps anti-*Trichinella.*

2. Utilisation selon la revendication 1, **caractérisée en ce que** l'on utilise un mélange comprenant un premier polypeptide tel que défini dans la revendication 1 et un second polypeptide comprenant les acides aminés 21-67 de la séquence SEQ ID NO: 4 ou une séquence présentant au moins 95% d'identité avec la séquence des acides aminés 21-67 de la séquence SEQ ID NO: 4 et le dit second polypeptide étant reconnu par des anticorps anti-*Trichinella.*

3. Polypeptide antigénique reconnu par des anticorps anti-*Trichinella,* **caractérisé en ce qu'**il est choisi parmi :
- un polypeptide chimérique comprenant :
(i) une ou plusieurs copies d'un premier polypeptide comprenant la séquence SEQ ID NO: 2 ou une séquence présentant au moins 95% d'identité avec la séquence SEQ ID NO: 2 et ledit polypeptide étant reconnu par des anticorps anti-*Trichinella,* et
(ii) une ou plusieurs copies d'un second polypeptide comprenant les acides aminés 21-67 de la séquence SEQ ID NO: 4 ou une séquence présentant au moins 95% d'identité avec la séquence des acides aminés 21-67 de la séquence SEQ ID NO: 4 et ledit polypeptide étant reconnu par des anticorps anti-*Trichinella,* éventuellement fusionnés à un ou plusieurs polypeptide(s) hétérologue(s) ;
- un polypeptide chimérique comprenant plusieurs copies d'un polypeptide comprenant la séquence SEQ ID NO: 2 ou une séquence présentant au moins 95% d'identité avec la séquence SEQ ID NO: 2 et ledit polypeptide étant reconnu par des anticorps anti-*Trichinella,* éventuellement fusionnés à un ou plusieurs polypeptide(s) hétérologue(s).

4. Polynucléotide codant pour un polypeptide antigénique selon la revendication 3.

5. Vecteur recombinant contenant un ou plusieurs polynucléotide(s) selon la revendication 4.

6. Cellule-hôte transformée par un vecteur recombinant selon la revendication 5.

7. Procédé de détection de la présence d'anticorps anti-*Trichinella* induits par l'infestation par *Trichinella,* dans un échantillon biologique, lequel procédé est **caractérisé en ce qu'**il comprend :
- la mise en contact dudit échantillon biologique avec un polypeptide tel que défini dans la revendication 1, un mélange de peptides tel que défini dans la revendication 2, ou un peptide chimérique tel que défini dans la revendication 3, dans des conditions permettant la formation d'un complexe antigène/anticorps avec les anticorps anti-*Trichinella* éventuellement présents dans ledit échantillon ;
- la détection, par tous moyens appropriés, du complexe antigène/anticorps éventuellement formé.

8. Nécessaire pour la détection de la présence d'anticorps anti-*Trichinella* induits par l'infestation par *Trichinella,* dans un échantillon biologique, **caractérisé en ce qu'**il comprend :
- un polypeptide, lequel polypeptide comprenant la séquence SEQ ID NO: 2 ou une séquence présentant au moins 95% d'identité avec la séquence SEQ ID NO: 2 et ledit polypeptide étant reconnu par des anticorps anti-*Trichinella,*
- un mélange de polypeptides comprenant un premier polypeptide tel que défini ci-dessus et un second polypeptide comprenant les acides aminés 21-67 de la séquence SEQ ID NO: 4 ou une séquence présentant au moins 95% d'identité avec la séquence des acides aminés 21-67 de la séquence SEQ ID NO: 4 et ledit second polypeptide étant reconnu par des anticorps anti-*Trichinella,* ou
- un polypeptide chimérique tel que défini dans la revendication 3, et
- des tampons et réactifs appropriés pour la constitution d'un milieu réactionnel permettant la formation d'un complexe antigène/anticorps.

9. Nécessaire selon la revendication 8, **caractérisé en ce que** le(s)dit(s) polypeptide(s) est (sont) immobilisé(s) sur un support solide.

## Patentansprüche

1. Verwendung eines antigenen Polypeptids als Reagenz zum Nachweis von Anti-*Trichinella*-Antikörpern, induziert durch Befall mit *Trichinella* in einer biologischen Probe, **dadurch gekennzeichnet, dass** das Polypeptid die Sequenz SEQ ID NO: 2 oder eine Sequenz umfasst, die mindestens 95 % Identität mit der Sequenz SEQ ID NO: 2 aufweist und wobei das Polypeptid von Anti-*Trichinella*-Antikörpern erkannt wird.

2. Verwendung nach Anspruch 1, **dadurch gekennzeichnet, dass** ein Gemisch verwendet wird, das ein erstes Polypeptid nach Anspruch 1 und ein zweites Polypeptid umfasst, umfassend die Aminosäuren 21-67 der Sequenz SEQ ID NO: 4 oder einer Sequenz, die mindestens 95 % Identität mit der Sequenz der Aminosäuren 21-67 der Sequenz SEQ ID NO: 4 aufweist und wobei das zweite Polypeptid von Anti-*Trichinella*-Antikörpern erkannt wird.

3. Antigenes Polypeptid, das von *Anti-Trichinella-*Antikörpern erkannt wird, **dadurch gekennzeichnet, dass** es ausgewählt ist aus:
- einem chimerischen Polypeptid, umfassend:
(i) eine oder mehrere Kopien eines ersten Polypeptids, umfassend die Sequenz SEQ ID NO: 2 oder eine Sequenz, die mindestens 95 % Identität mit der Sequenz SEQ ID NO: 2 aufweist und wobei das zweite Polypeptid von *Anti-Trichinella-*Antikörpern erkannt wird, und
(ii) eine oder mehrere Kopien eines zweiten Polypeptids, umfassend die Aminosäuren 21-67 der Sequenz SEQ ID NO: 4 oder einer Sequenz, die mindestens 95 % Identität mit der Sequenz der Aminosäuren 21-67 der Sequenz SEQ ID NO: 4 aufweist und wobei das zweite Polypeptid von Anti-*Trichinella*-Antikörpern erkannt wird, die eventuell mit einem oder mehreren heterologen Polypeptiden fusioniert haben,
- einem chimerischen Polypeptid, umfassend mehrere Kopien eines Polypeptids, umfassend die Sequenz SEQ ID NO: 2 oder eine Sequenz, die mindestens 95 % Identität mit der Sequenz SEQ ID NO: 2 aufweist und wobei das Polypeptid von Anti-*Trichinella*-Antikörpern erkannt wird, die eventuell mit einem oder mehreren heterologen Polypeptiden fusioniert haben.

4. Polynukleotid, codierend für ein antigenes Polypeptid nach Anspruch 3.

5. Rekombinanter Vektor, enthaltend ein oder mehrere Polynukleotide nach Anspruch 4.

6. Wirtszelle, transformiert von einem rekombinanten Vektor nach Anspruch 5.

7. Verfahren zum Nachweis des Vorhandenseins von Anti-*Trichinella*-Antikörpern in einer biologischen Probe, wobei das Verfahren **dadurch gekennzeichnet ist, dass** es umfasst:
- das Inkontaktversetzen der biologischen Probe mit einem Polypeptid nach Anspruch 1, einem Polypeptidgemisch nach Anspruch 2, oder einem chimerischen Peptid nach Anspruch 3 unter Bedingungen, die die Bildung eines Komplexes Antigen/Antikörper mit eventuell in der Probe vorhandenen Anti-*Trichinella*-Antikörpern erlauben,
- den Nachweis mit allen geeigneten Mitteln des eventuell gebildeten Komplexes Antigen/Antikörper.

8. Kit für den Nachweis des Vorhandenseins von Anti-*Trichinella*-Antikörpern, induziert durch Befall mit *Trichinella* in einer biologischen Probe, **dadurch gekennzeichnet, dass** es umfasst:
- ein Polypeptid, wobei das Polypeptid die Sequenz SEQ ID NO: 2 oder eine Sequenz umfasst, die mindestens 95 % Identität mit der Sequenz SEQ ID NO: 2 aufweist und wobei das Polypeptid von Anti-*Trichinella*-Antikörpern erkannt wird,
- ein Polypeptidgemisch, umfassend ein erstes, wie oben definiertes Polypeptid und ein zweites Polypeptid, umfassend die Aminosäuren 21-67 der Sequenz SEQ ID NO: 4 oder eine Sequenz, die mindestens 95 % Identität mit der Sequenz der Aminosäuren 21-67 der Sequenz SEQ ID NO: 4 aufweist und wobei das zweite Polypeptid von Anti-*Trichinella*-Antikörpern erkannt wird,
- ein chimerisches Polypeptid nach Anspruch 3, und
- Puffer und Reagenzien, die für die Bildung eines Reaktionsmilieus geeignet sind, das die Bildung eines Komplexes Antigen/Antikörper erlaubt.

9. Kit nach Anspruch 8, **dadurch gekennzeichnet, dass** das/die Polypeptid(e) auf einer festen Unterlage immobilisiert ist/sind.

## Claims

1. Use of an antigenic polypeptide as a reactant for the detection of anti-*Trichinella* antibodies induced by infestation by *Trichinella* in a biological sample, **characterised in that** said polypeptide comprises the sequence SEQ ID NO: 2 or a sequence having at least 95% identity with the sequence SEQ ID NO: 2 and said polypeptide being recognised by anti-*Trichinella* antibodies.

2. Use according to claim 1, **characterised in that** a mixture comprising a first polypeptide as defined in claim 1 and a second polypeptide comprising the amino acids 21-67 of the sequence SEQ ID NO: 4 or a sequence having at least 95% identity with the sequence of the amino acids 21-67 of the sequence SEQ ID NO: 4 is used, and said second polypeptide being recognized by anti-*Trichinella* antibodies.

3. Antigenic polypeptide recognized by anti-*Trichinella* antibodies, **characterised in that** said polypeptide is chosen from:
- a chimeric polypeptide comprising:
(i) one or more copies of a first polypeptide comprising the sequence SEQ ID NO: 2 or a sequence having at least 95% identity with the sequence SEQ ID NO: 2, said polypeptide being recognised by anti-*Trichinella* antibodies, and
(ii) one or more copies of a second polypeptide comprising the amino acids 21-67 of the sequence SEQ ID NO: 4 or a sequence having at least 95% identity with the sequence of the amino acids 21-67 of the sequence SEQ ID NO: 4, said polypeptide being recognized by *anti-Trichinella* antibodies, optionally fused with one or more heterologous polypeptides;
- a chimeric polypeptide comprising a plurality of copies of a polypeptide comprising the sequence SEQ ID NO: 2 or a sequence having at least 95% identity with the sequence SEQ ID NO: 2, said polypeptide being recognised by *anti-Trichinella* antibodies, optionally fused with one or more heterologous polypeptides.

4. Polynucleotide coding for an antigenic polypeptide according to claim 3.

5. Recombinant vector containing one or more polynucleotides according to claim 4.

6. Host cell transformed by a recombinant vector according to claim 5.

7. Method for detection of the presence of anti-*Trichinella* antibodies induced by infestation by *Trichinella,* in a biological sample, which method is **characterised in that** said method involves:
- putting said biological sample in contact with a polypeptide as defined in claim 1, a mixture of peptides as defined in claim 2, or a chimeric peptide as defined in claim 3, in conditions allowing the formation of an antigen/antibody complex with the anti-*Trichinella* antibodies possibly present in said sample;
- the detection, by any suitable means, of the antigen/antibody complex possibly formed.

8. Kit for the detection of the presence of anti-*Trichinella* antibodies induced by infestation by *Trichinella,* in a biological sample, **characterised in that** said kit comprises:
- a polypeptide, which polypeptide comprises the sequence SEQ ID NO: 2 or a sequence having at least 95% identity with the sequence SEQ ID NO: 2, said polypeptide being recognised by anti-*Trichinella* antibodies,
- a mixture of polypeptides comprising a first polypeptide as defined above and a second polypeptide comprising the amino acids 21-67 of the sequence SEQ ID NO: 4 or a sequence having at least 95% identity with the sequence of the amino acids 21-67 of the sequence SEQ ID NO: 4, and said second polypeptide being recognised by anti-*Trichinella* antibodies, or
- a chimeric polypeptide as defined in claim 3, and
- buffers and reactants suitable for forming a reaction medium allowing the formation of an antigen/antibody complex.

9. Kit according to claim 8, **characterised in that** said polypeptide (s) are immobilised on a solid substrate.


### SEQUENCE LISTING

<110> AGENCE NATIONALE CHARGEE DE LA SECURITE SANITAIRE DE
   L'ALIMENTATION, DE L'ENVIRONNEMENT ET DU TRAVAIL
   ZOCEVIC, Aleksandar
   BALDISSERA, Giovani
   LACOUR, Sandrine A.
   MACE, Pauline
   VALLEE, Isabelle
   BOIREAU, Pascal
<120> Antigènes polypeptidiques de Trichinella, et leurs applications
<130> MJP/11-F1159-7 WO
<150> FR 10 00660
   <151> 2010-12-17
<160> 4
<170> PatentIn version 3.5
<210> 1
   <211> 398
   <212> DNA
   <213> Trichinella spiralis
<220>
   <221> CDS
   <222> (34)..(351)
<400> 1
   gggtcagttt tagcagcttt tatcttcttt ttc atg gca gtt atg cct gaa atc        54
                                        Met Ala Val Met Pro Glu Ile
                                        1               5
   aat gcg gat ttg agt cca ttg gaa gaa gcc caa agt tac ata tac caa        102
   Asn Ala Asp Leu Ser Pro Leu Glu Glu Ala Gln Ser Tyr Ile Tyr Gln
           10                  15                  20
   tct gat ttg caa agc ggt aaa ggt cat ttc cgc aga gtt ctc gat ata        150
   Ser Asp Leu Gln Ser Gly Lys Gly His Phe Arg Arg Val Leu Asp Ile
       25                  30                  35
   agc gat gtc gac aca agt gac gga tta tcc tta acg ata gac gct ctt        198
   Ser Asp Val Asp Thr Ser Asp Gly Leu Ser Leu Thr Ile Asp Ala Leu
   40                  45                  50                  55
   cca act aca tgt cct gtg tca tca gaa atg act caa gat caa gtg tat        246
   Pro Thr Thr Cys Pro Val Ser Ser Glu Met Thr Gln Asp Gln Val Tyr
                   60                  65                  70
   tca gat gag tgc ccc gtc acc aga gag gaa tat gac gaa ata gaa tgc        294
   Ser Asp Glu Cys Pro Val Thr Arg Glu Glu Tyr Asp Glu Ile Glu Cys
               75                  80                  85
   cat ttg aag ctt gac cat tct aaa act ggc caa att gaa tgt aca tat        342
   His Leu Lys Leu Asp His Ser Lys Thr Gly Gln Ile Glu Cys Thr Tyr
           90                  95                  100
   tat gga cat taaactatga gaataaagtg atttaatgaa aaaaaaaaaa aaaaaaa         398
   Tyr Gly His
       105
<210> 2
   <211> 106
   <212> PRT
   <213> Trichinella spiralis
<400> 2
   Met Ala Val Met Pro Glu Ile Asn Ala Asp Leu Ser Pro Leu Glu Glu
   1               5                   10                  15
   Ala Gln Ser Tyr Ile Tyr Gln Ser Asp Leu Gln Ser Gly Lys Gly His
               20                  25                  30
   Phe Arg Arg Val Leu Asp Ile Ser Asp Val Asp Thr Ser Asp Gly Leu
           35                  40                  45
   Ser Leu Thr Ile Asp Ala Leu Pro Thr Thr Cys Pro Val Ser Ser Glu
       50                  55                  60
   Met Thr Gln Asp Gln Val Tyr Ser Asp Glu Cys Pro Val Thr Arg Glu
   65                  70                  75                  80
   Glu Tyr Asp Glu Ile Glu Cys His Leu Lys Leu Asp His Ser Lys Thr
                   85                  90                  95
   Gly Gln Ile Glu Cys Thr Tyr Tyr Gly His
               100                 105
<210> 3
   <211> 339
   <212> DNA
   <213> Trichinella spiralis
<220>
   <221> CDS
   <222> (1)..(201)
<400> 3
   atg ttc atc acg ttt atc ttt ctt gct aac ata ctg ctt ctt gtg caa        48
   Met Phe Ile Thr Phe Ile Phe Leu Ala Asn Ile Leu Leu Leu Val Gln
   1               5                   10                  15
   cca tcg gaa gca tat cgt ggt cac acc aac gat gaa att cga ttg atg        96
   Pro Ser Glu Ala Tyr Arg Gly His Thr Asn Asp Glu Ile Arg Leu Met
               20                  25                  30
   gat gag tgt agc gat gaa cca tac ata cga gaa cac ttg ggg gaa gat       144
   Asp Glu Cys Ser Asp Glu Pro Tyr Ile Arg Glu His Leu Gly Glu Asp
           35                  40                  45
   gat tat atg agt tta att gat gcg tgc gtt gaa gaa cga ctt gga cga       192
   Asp Tyr Met Ser Leu Ile Asp Ala Cys Val Glu Glu Arg Leu Gly Arg
       50                  55                  60
   aga gtt gca tgaagaatat aagaaaagct atcaagaatt gttcattttc               241
   Arg Val Ala
   65
   aagcgacaat tttatttatg aaatgaattt attgaaaaat gaaaatctgt tacagtattc     301
   gtaataaata gctatgcagt aaaaaaaaaa aaaaaaaa                             339
<210> 4
   <211> 67
   <212> PRT
   <213> Trichinella spiralis
<400> 4
   Met Phe Ile Thr Phe Ile Phe Leu Ala Asn Ile Leu Leu Leu Val Gln
   1               5                   10                  15
   Pro Ser Glu Ala Tyr Arg Gly His Thr Asn Asp Glu Ile Arg Leu Met
               20                  25                  30
   Asp Glu Cys Ser Asp Glu Pro Tyr Ile Arg Glu His Leu Gly Glu Asp
           35                  40                  45
   Asp Tyr Met Ser Leu Ile Asp Ala Cys Val Glu Glu Arg Leu Gly Arg
       50                  55                  60
   Arg Val Ala
   65